# EUROPEAN PATENT APPLICATION

(11) **EP 3 906 899 A1**
(43) Date of publication of application: **10.11.2021**
(21) Application number: 21181556.8
(22) Date of filing: 12.01.2017
(51) Int. Cl.: A61F 2/82, A61F 2/01, A61B 17/221

(54) **IMPLANTABLE DEVICES CAPABLE OF SELECTIVE DEGRADATION**

(30) Priority: 12.01.2016 US 201662277713 P; 11.01.2017 US 201715403453
(62) Divisional of application: 17704110.0
(71) Applicant: W.L. Gore & Associates Inc., Newark, DE 19711 (US)
(72) Inventor: CULLY, Edward, H., 551 Paper Mill Road, Newark, Delaware, 19711 (US); VECCHIO, Christopher, J., 551 Paper Mill Road, Newark, Delaware, 19711 (US)
(74) Representative: HGF

(57) **Abstract**

Implantable medical devices that contain at least one region that is selectively degradable by electrolytic corrosion are provided. The electrolytic corrosion of the medical device is initiated by the formation of an electrolytic cell that can be activated wirelessly at a designated point in time. The medical device incorporates at least one section or region that is designed to be predisposed to structural failure. The medical device contains a cathode region, a sacrificial anode region, which will undergo degradation, and an antenna region. Electrolytic degradation of a sacrificial anode region may cause a de-anchoring of the medical device or a reconfiguration of the medical device from a first configuration to a second configuration. Alternatively, electrolytic degradation may precipitate the absorption of the medical device. In another embodiment, electrolytic protection may be employed to preserve an implanted device until such a time that its corrosion and subsequent absorption is desired.

## Description

### FIELD

The present disclosure relates generally to implantable medical devices, and more specifically, to a medical device that contains at least one region that can be selectively degraded by electrolytic corrosion.

### BACKGROUND

A variety of medical devices have been developed for implantation within an anatomy or body (e.g., a human body). Many such devices are implantable within a body lumen (*e.g.,* the vasculature and/or gastrointestinal tract ("GI tract") of a human body). For instance, devices like stents, grafts, and stent-grafts may be implanted within the vasculature and/or GI tract of a human body to reinforce, replace, and/or bridge a damaged, unhealthy, or otherwise diseased portion of a body lumen. These devices may thus, in certain instances, guide blood and/or other fluids through a lumen defined by a cylindrical interior surface. During implantation, it is often necessary to anchor such devices in place, so that they will not migrate away from a damaged or diseased portion of the anatomy they are intended to repair.

Once deployed to the desired position within a patient, the ongoing efficacy of implantable devices can often depend on their ability to remain in an approximately fixed position relative to the surrounding tissue. For example, an occlusion device implanted to occlude or close an aperture should maintain its proper position relative to the tissue surrounding the aperture, or it may fail to close the aperture. Similarly, a stent graft device deployed in the location of a stricture should remain in the location of the lumen stricture to create or enlarge an open passageway for fluid flow.

In addition, it may be desirable for the medical device to be removed once the intended therapy or treatment is completed. Removal of such devices may be difficult due to tissue growth into and around the medical device. Thus, there exists a need in the art for a medical device that can be used in intraluminal or transluminal applications for the fully intended term of therapy and which can be removed with minimal trauma to the surrounding tissue and to the patient and without invasive or endoscopic procedures.

### SUMMARY

One embodiment relates to an implantable device that has a cathode region, a sacrificial anode region, and an antenna region. The implantable device may include at least one predetermined failure region susceptible to electrolytic degradation. Electrolytic degradation of the anode region and/or the predetermined failure region transforms the implantable device from a first configuration to a second configuration. The electrolytic degradation is initiated by the formation of an electrolytic cell that is formed when the antenna region remotely receives energy from an external transmitter device. In one embodiment, selective electrolytic corrosion by the formation of an electrolytic cell may be used to adjust the medical device from a first configuration to a second configuration. As one example, electrolytic degradation may be used to adjust the diameter of an implanted medical device, such as, for example, an adjustable diameter stent. As another example, the first configuration may be an anchored configuration where the medical device is anchored to a lumen and the second configuration may be a non-anchored configuration. Once de-anchored from the lumen, the device may be non-invasively removed, such as by passage through the digestive tract.

A second embodiment relates to an implantable device that has a cathode region, a sacrificial anode region, and a piezoelectric receiver region. A bridge rectifier may be used to increase power transfer efficiency. Electrolytic degradation of the sacrificial anode region transforms the implantable device from a first configuration to a second configuration. Electrolytic degradation is initiated upon the formation of an electrolytic cell, which is formed when the piezoelectric receiver region receives acoustic energy from an external transmitter device and converts the acoustic energy to electrical energy. In one embodiment, selective electrolytic corrosion by the formation of an electrolytic cell may be used to adjust a medical device from a first configuration to a second configuration. As one example, electrolytic degradation may be used to adjust the diameter of an implanted medical device, such as, for example, an adjustable diameter stent. As another example, the first configuration may be an anchored configuration where the medical device is anchored to a lumen and the second configuration is a non-anchored configuration. Once de-anchored from the lumen, the device may be non-invasively removed, such as by passage through the digestive tract.

A third embodiment relates to a method for remotely reconfiguring a device from a first configuration to a second configuration by receiving electrical energy from an external transmitter device to form an electrolytic cell. The formation of the electrolytic cell causes electrolytic degradation of a sacrificial anode region in the device. The implantable device may include at least one predetermined failure region susceptible to electrolytic degradation. For example, a region may be made susceptible to electrolytic degradation by making it thinner than surrounding regions or by insulating surrounding regions and leaving the susceptible region uninsulated.

A fourth embodiment relates to an implantable device that includes a cathode region and a sacrificial anode region. An electrolytic cell is formed between the cathode region and the sacrificial anode region when an antenna region remotely receives energy from an external transmitter device. A rectification circuit may be positioned adjacent to the antenna region to convert the electrical energy to direct current voltage. The formation of the electrolytic cell induces electrochemical corrosion of the anode region, which transforms the device from a first configuration to a second configuration. In one embodiment, the implantable device has a first configuration as a vascular filter where emboli blocking elements are positioned in the blood stream, and a second configuration as a stent where the emboli blocking elements are removed from the bloodstream after embolic protection is no longer required.

A fifth embodiment relates to an implantable device that includes a cathode region and a sacrificial anode region. An electrolytic cell is formed between the cathode region and the sacrificial anode region when an antenna region remotely receives energy from an external transmitter device. A rectification circuit may be positioned remotely, e.g., adjacent to the antenna region, to convert the electrical energy to direct current voltage. The formation of the electrolytic cell induces electrochemical corrosion of the anode region, which transforms the device from a first configuration to a second configuration. In one embodiment, the implantable device has a first configuration as a tissue defect closure device, which effectively and securely closes a defect, such as an atrial septal defect or ventricular septal defect, and a second configuration where the structure of the defect closure device is compromised, thereby transforming the mechanical properties of the device from rigid (as implanted) to soft and conformable (e.g., after sufficient tissue ingrowth has occurred) to maintain the device in position. In this manner, the device may work acutely to close the defect, then, at the discretion of a clinician, for example, be transformed to a pliable, conformable, long-term implant that does not abrade or irritate the host tissue with which it is in contact

A sixth embodiment relates to an implantable device fabricated wholly or in part from a material, which would naturally dissolve in the body absent a current impressed upon it. The device may include at least one dissolvable region, an anode region, and an antenna region. The antenna region remotely receives energy from an external transmitter device. The energy is used to maintain the dissolvable region of the device at a negative voltage potential with respect to the anode region. When the medical device is no longer needed, the supply of energy is discontinued and corrosion begins to dissolve the dissolvable region(s). In at least one embodiment, the medical device completely dissolves. Alternatively, the medical device is dissolved to a point where it can be naturally expelled by the body.

A seventh embodiment relates to an implantable device that includes a cathode region and an antenna region. The frame of the device has an electrically conductive, corrosion resistant core and an electrochemically degradable outer surface. An electrolytic cell is formed between the cathode region and the frame (which acts as the anode of the electrolytic cell) when energy is remotely received by the antenna region from an external transmitter device. The formation of the electrolytic cell degrades the outer surface of the frame, thereby compromising the structural integrity of the frame. As a result, a structurally stiff frame or frame member may thereby be transformed from a rigid device into a flexible or conformable structural element.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a schematic illustration of a stent adjusting from a first diameter to a second diameter through electrolytic degradation of a wire helically wrapped around the stent according to one embodiment;
FIG. 2 is a schematic illustration of a stent constrained to a first diameter by a wire helically wrapped around the stent and piezoelectric circuitry connected thereto that induces electrolytic corrosion of a sacrificial anode region in accordance with an embodiment;
FIG. 2A is a schematic illustration of a variation of the circuitry depicted in FIG. 2 but which utilizes a bridge rectifier to increase power transfer efficiency in accordance with an embodiment;
FIG. 3 is a schematic illustration of a variation on circuitry which uses the frame of the stent as a receiving antenna according to another embodiment;
FIG. 4 is a schematic illustration of an implantable device containing spring force anchor wings having a narrowed portion therein that is subject to electrolytic degradation according to an embodiment;
FIG. 5A is a schematic illustration of an embolic filter with a sacrificial region which holds the filter closed at its apex in accordance with an embodiment;
FIG. 5B is a schematic illustration of an unobstructed stent-graft that is formed upon the degradation of the sacrificial region in the embolic filter of FIG. 5A in accordance with an embodiment;
FIG. 6A is a schematic illustration of an occluder having a plurality of thinned areas in the frame of the occluder according to an embodiment;
FIG. 6B is a schematic illustration of the occluder of FIG. 6A having portions of the frame removed by electrochemical degradation in accordance with one embodiment;
FIG. 6C is an enlarged view of the thinned areas on the frame of the occluder depicted in FIG. 6A in accordance with an embodiment;
FIG. 6D is an enlarged view of the electrochemically degraded regions of the frame of the occluder depicted in FIG. 6B according to one embodiment;
FIG. 7 is a schematic illustration of an implantable medical device capable of being completely degraded by electrochemical corrosion in accordance with an embodiment;
FIG. 8 is a schematic illustration of an implantable medical device fabricated from a material which would dissolve in the body absent energy supplied to a receiving coil connected to the medical device according to one embodiment;

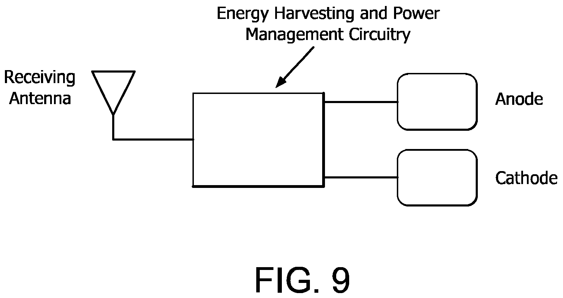

FIG. 9 is a schematic illustration generally depicting circuitry for use herein;
FIG. 10 is a schematic illustration of a medical device adjusting from a first configuration to a second configuration through electrolytic degradation of a constraining member wrapped around the medical device according to one exemplary embodiment; and
FIG. 11 is a schematic illustration of rendering of a medical device conformable through the electrochemical degradation of the outer surface of the medical device in accordance with an embodiment.

### DETAILED DESCRIPTION

Persons skilled in the art will readily appreciate that various aspects of the present disclosure can be realized by any number of methods and apparatus configured to perform the intended functions. It should also be noted that the accompanying figures referred to herein are not necessarily drawn to scale, but may be exaggerated to illustrate various aspects of the present disclosure, and in that regard, the drawing figures should not be construed as limiting.

The present invention is directed to implantable medical devices that contain at least one region that is selectively degradable by electrolytic corrosion. The electrolytic corrosion is initiated by the formation of an electrolytic cell that can be activated wirelessly at a designated point in time. Additionally, the medical device can incorporate one or more section(s) or region(s) therein that are designed to be predisposed to structural failure. The medical device may contain a cathode region, an anode region that will undergo degradation, and an antenna region. Electrolytic degradation of the anode region may cause, for example, a de-anchoring of the medical device, a reconfiguration of the medical device from a first configuration to a second configuration, or it may precipitate the absorption of the medical device. Alternatively, electrolytic protection may be employed to preserve an implanted device until such a time that its corrosion and subsequent absorption is desired. It is to be appreciated that the terms electrolytic protection, cathodic protection, and impressed current cathodic protection as used herein refer to the application of a voltage potential to a structure in order to inhibit corrosion of the structure.

In one embodiment, selective electrolytic corrosion by the formation of an electrolytic cell may be used to adjust the medical device from a first configuration to a second configuration. As one example, electrolytic degradation may be used to adjust the diameter of an implanted medical device, such as, for example, an adjustable diameter stent. The stent may be any conventional tubular or radially expandable stent having a generally flexible frame and an opening extending therethrough. The frame of the stent may be formed of one or more elongate member (e.g., a wire) that has been helically wrapped into a tubular form. Additionally, the stent may be covered or partially covered with a cover material. It is to be appreciated that cover materials may be chosen and configured so as to confine and trap any degradation products produced during the degradation process. Stents described herein may be used in a wide variety of different anatomies, implant sites (e.g., body lumens, organs, cavities, and the like) and types of im plementations.

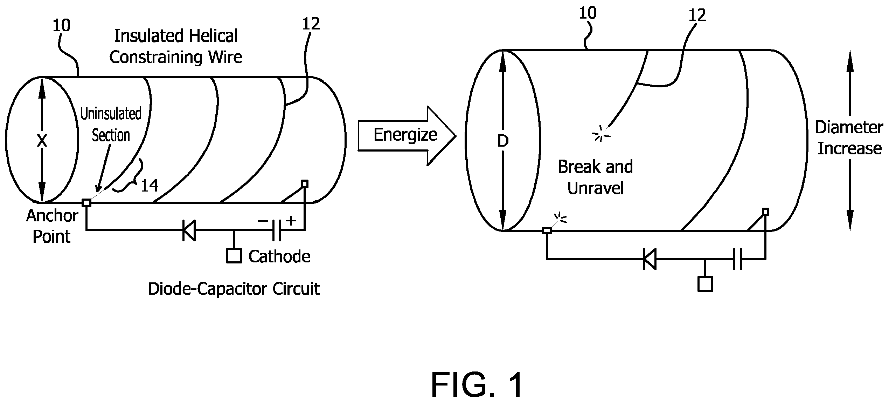

Turning to FIG. 1, a constraining member 12 wrapped helically around an outer surface of the stent 10 restrains the stent 10 to a diameter (X) (e.g., first configuration) that is less than its maximum diameter. "Outer surface" as used in connection with this embodiment is meant to denote the most external surface of the stent 10 that faces and/or is in contact with the wall of a lumen when implanted. Additionally, the term "lumen", as used herein, is meant to denote a hollow tubular structure, such as, for example, an artery, intestine, duct, or tract. The constraining member 12 may be formed in whole or in part of at least one material that is susceptible to electrolytic degradation, such as, but not limited to, steel, stainless steel, nitinol, copper, zinc, aluminum, nickel, tungsten, and titanium. In one exemplary embodiment, the constraining member 12 is insulated over its length with the exception of one or more sacrificial regions, where it is uninsulated and predisposed to electrochemical degradation. The insulation may be a biocompatible coating (e.g., sprayed, dipped or deposited onto the surface of the device) or a covering that is applied by wrapping or adhering the cover to the surface of the device.

In the embodiment depicted in FIG. 1, the constraining member 12 acts as the antenna in the diode-capacitor circuit. Energy wirelessly provided to the antenna from an external source (not illustrated) is rectified by the diode which results in the generation of a DC electrical potential across the capacitor with a polarity (indicated by the "+" and "-" labels). The electrically conductive elements connected to the positive side of the capacitor (labeled "+") become positively charged with respect to the electrically conductive elements that are connected to the negative side of the capacitor (labeled "-"). When implanted in a body, any uninsulated section of the positively charged elements will be exposed to surrounding tissues and fluids and will form the "anode". Similarly, any uninsulated section of the negatively charged elements will be exposed to surrounding tissues and fluids in the body and will form the "cathode". It is desirable for the cathode section to have as large a surface area as possible in order to reduce electrical resistance and maximize the electrolytic effects. As a result, all or substantially all, of the elements electrically connected to the cathode may be exposed and/or uninsulated.

When the stent 10 is energized, corrosion occurs over the uninsulated and/or exposed anode surfaces. Therefore, it is desirable to expose only the sections or regions in which degradation and mechanical failure are intended to occur. Limiting the area of the exposed anode region also serves to accelerate the degradation of the targeted, sacrificial region. In this manner energy wirelessly provided to the antenna (i.e., constraining member 12) from an external source creates an electrolytic cell and induces electrochemical corrosion at the exposed, sacrificial anode region 14. The antenna is activated remotely, and does need not to be in direct physical contact with the energy source.

Energy is provided until the sacrificial region 14 has corroded to an extent that the sacrificial region 14 breaks and releases the stent 10 from its constrained configuration to an expanded configuration. The degradation and subsequent release of the constraining member 12 permits the stent 10 to expand to its full (or substantially full) diameter (D) (e.g., second configuration) without the use of any invasive techniques. The fully expanded configuration of the stent 10 may anchor the medical device in a lumen. Alternatively, the expansion of the stent may cause anchors (not illustrated) affixed to the stent to engage with the lumen wall to anchor the stent therein. The expansion of the stent may be initiated in order to increase blood flow or to compensate for stenosis in the stent or lumen.

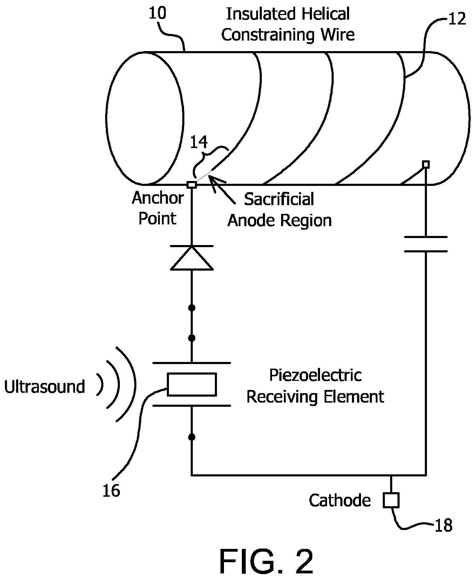

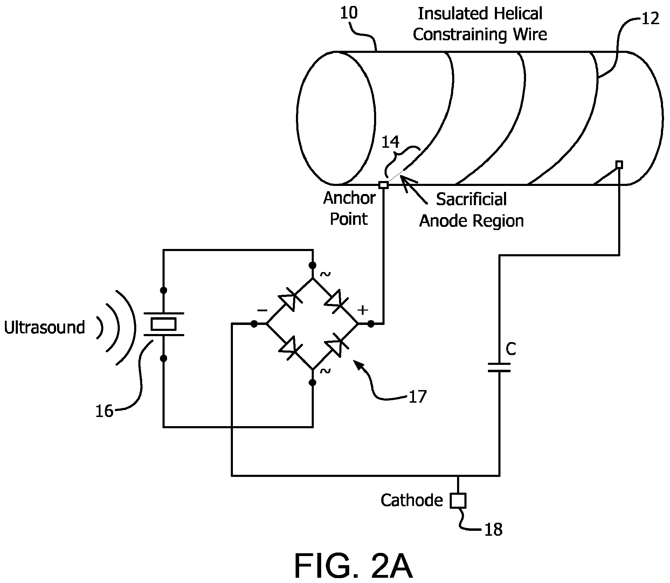

In another embodiment depicted in FIG. 2, the stent 10 has a piezoelectric receiving element 16, a cathode 18, and a sacrificial anode region 14. The piezoelectric receiving element 16 receives acoustic energy from an external transmitting device (not shown) and converts the acoustic energy to electrical energy, thereby forming an electrolytic cell. As with the embodiment described above, the energy provided to the constraining member 12 electrolytically degrades the sacrificial anode region 14 until the constraining member 12 breaks and releases the stent 10 from its constrained configuration (e.g., first position) to an expanded configuration (e.g., second position).

FIG. 2A is a schematic illustration of a variation of the circuitry depicted in FIG. 2 with the exception that a bridge rectifier 17 is used to increase power transfer efficiency. The bridge rectifier utilizes both halves of the induced alternating current (AC) waveform and thereby increases the effective direct current (DC) voltage and the electrical power applied to the electrolytic cell. It is to be appreciated that various circuit configurations and manners of energy harvesting and electrical power delivery to the electrolytic cell may be envisioned and the embodiments described herein should not be construed as limiting. For example, circuitry could be designed to store energy over a period of time and then energize the electrolytic cell when triggered by an external signal or once a predetermined amount of energy has been stored. Energy to energize the electrolytic cell may be gathered in whole or in part from kinetic, electromagnetic, thermal, infrared, bioelectric, photoelectric, electrochemical, or other sources or a combination of such sources. Tuned or resonant power transfer techniques may also be used to improve the efficiency of the wireless energy transmission.

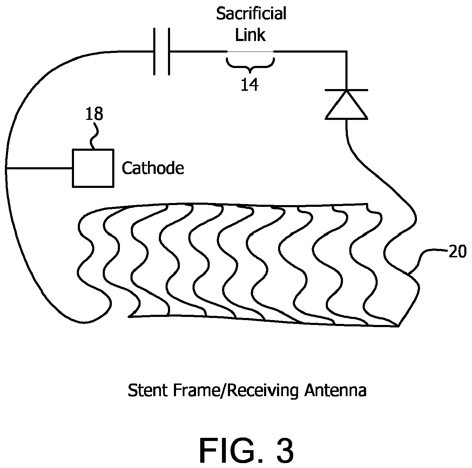

In some embodiments, the frame 20 of a stent may act as the receiving antenna. FIG. 3 depicts one example of a stent frame being used as a receiving antenna. Similar to the embodiments discussed above, energy from an external source is provided to the stent frame 20, thereby creating an electrolytic cell that induces electrochemical corrosion at the exposed, sacrificial anode region (link) 14.

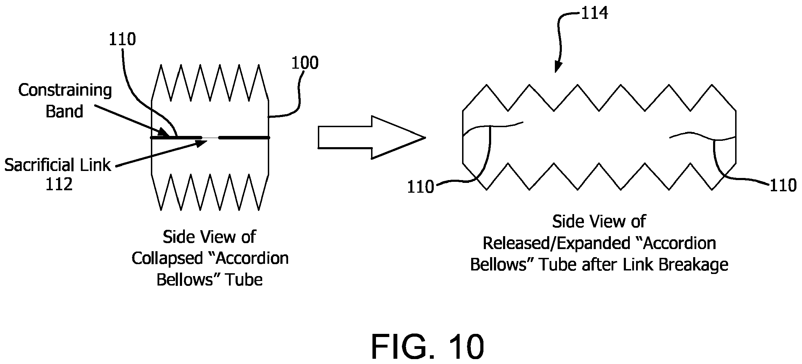

In another exemplary embodiment, the sacrificial anode region may act as a link that holds a scissor or accordion structure in a retracted or extended position. Turning to FIG. 10, a constraining member 110 wrapped around an outer surface of the medical device 100 restrains the medical device 100 in a constrained or first configuration. The constraining member 110 contains at least one sacrificial link 112 therein. Energy is provided to the sacrificial link 112 until the link has corroded to an extent that the constraining member 110 breaks and releases the medical device 100 from its first, constrained configuration to a second, expanded configuration 114. The degradation and subsequent breakage of the constraining member 112 permits the medical device to expand to a longer, narrower configuration.

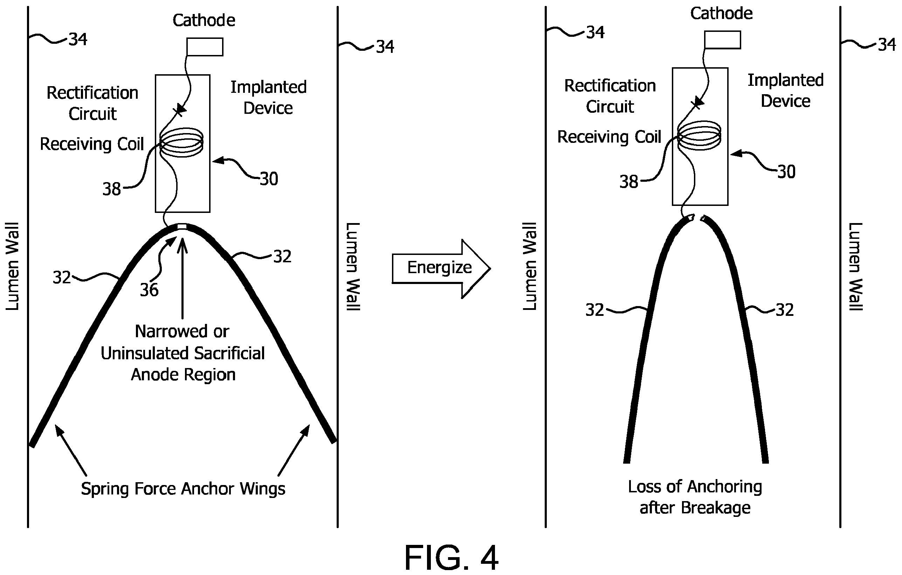

In a further exemplary embodiment, the formation of an electrolytic cell may be used to de-anchor a medical device from a lumen. FIG. 4 depicts an implantable medical device 30 with spring force anchor wings 32 that anchor the medical device within a lumen 34. The anchor wings 32 may have therein at least one narrowed region 36 that is susceptible to electrochemical degradation. An electrolytic cell is formed when the antenna region 38 (i.e., receiving coil) remotely receives electrical energy from an external transmitter device (not illustrated). The formation of the electrolytic cell induces electrochemical corrosion of the anchor wings 32. Because the narrowed region 36 has a smaller cross section than the remaining portion of the anchor wings 32, the narrowed region 36 is the first, and/or the only, portion of the anchor wings 32, to fully degrade. In addition, the remaining portion of the anchor wings 32 may be partially or completely insulated over its length with the exception of the narrowed region 36 in order to preferentially degrade the narrow region 36.

The degradation of the narrowed or uninsulated sacrificial anode region 36 of the anchor wings 32 causes the anchor wings 32 to fail in that once the narrow region 36 is electrochemically degraded and breaks, the anchor wings 32 are no longer able to apply the anchoring spring force necessary to hold the medical device 30 in the lumen 34. As a result, the medical device 30 is de-anchored from the lumen 34. Such a de-anchoring of the medical device 30 permits the medical device 30 to be non-invasively removed, such as via passage through the digestive tract. It is to be appreciated that regions susceptible to electrolytic degradation may also be created by electrically insulating a positively charged section(s) of the circuit with the exception of the regions, which are intended to degrade. A combination of narrowing and selective insulation may be used to predispose section(s) of the anchor wings 32 to electrochemical degradation.

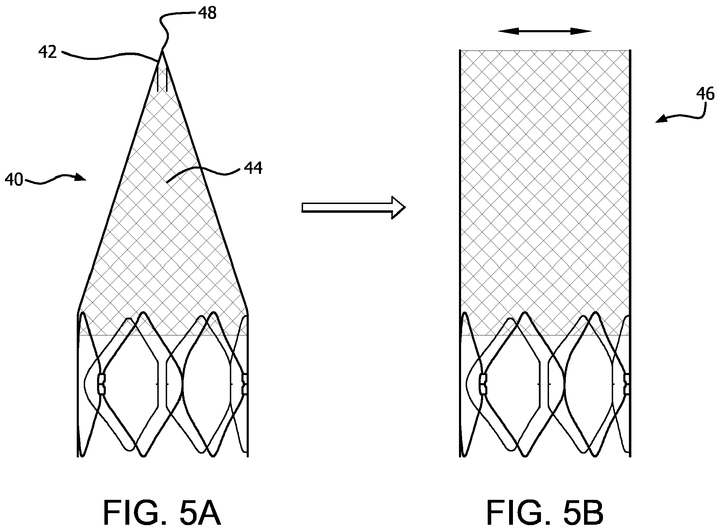

In yet another embodiment, electrolytic degradation may be used to transform a medical device having one purpose into a second medical device having a second purpose. As one non-limiting example, an embolic filter may be electrochemically degraded and transformed into a stent-graft *in situ.* FIG. 5A depicts an embolic filter 40 containing a sacrificial region 42 that holds the filter 40 closed at its apex 48. The sacrificial region 42 forms the anode of an electrochemical cell along with a cathode region (not shown). Similar to the embodiments previously described, energy wirelessly received by an antenna energizes the cell and induces electrolytic degradation of the sacrificial region 42. Degradation of the sacrificial region 42 permits the wall 44 of the embolic filter 40 to break open at the apex 48 and form an open tube, such as an unobstructed stent-graft 46 as generally depicted in FIG. 5B. The stent graft 46 has a generally tubular configuration and an unobstructed passageway to permit the passage of fluid therethrough. The open configuration of the stent-graft 46 may anchor the stent-graft 46 in a lumen. In this embodiment, the use of selective electrochemical degradation permits a physician to transform an embolic filter into a stent-graft at an appropriate time and without any invasive technology.

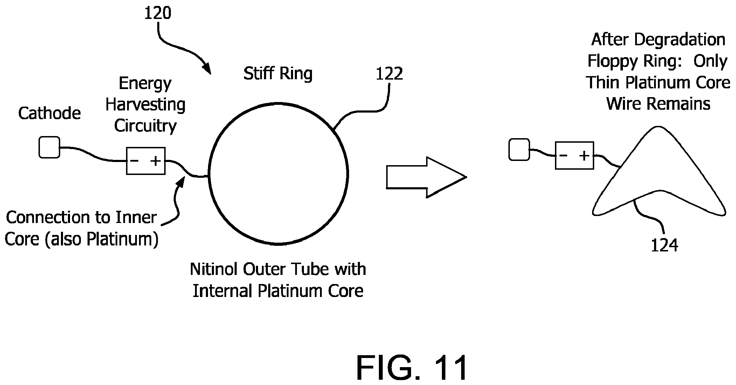

In another embodiment, a medical device may be rendered conformable through the electrochemical degradation of a portion of the medical device. One non-limiting embodiment is depicted generally in FIG. 11. FIG. 11 depicts a device formed of a drawn filled tube having an electrically conductive core and an outer surface. The core may be formed of a corrosion-resistant conductive material such as platinum, iridium, or gold, and alloys thereof and combinations thereof. The outer surface may be formed of one or more conductive electrochemically degrading material such as any of those described above.

The conductive, corrosion-resistant core facilitates an extensive electrolytic degradation of the outer surface as it will continue to maintain electrical conductivity of the device via the electrically conductive (corrosion-resistant) core, even after a section or sections of the outer surface have been completely eroded. In such an embodiment, the core may be thin, flexible, and able to maintain an electrical connection between sections of the device, but is not able to contribute significantly to the device's strength or stiffness. The outer material, in contrast, maintains the strength and stiffness of the device until the material is degraded.

Still referring to FIG. 11, energy may be transmitted to the outer surface 122 of the device 120 from a remote energy source to erode the material forming the outer surface 122. Once the outer surface 122 has been electrochemically degraded, the corrosion-resistant inner core 124 is exposed. Because the inner core 124 lacks structural integrity, and the entirety (or substantially the entirety) of the outer surface 122 has been eroded, the medical device becomes conformable over its entire surface.

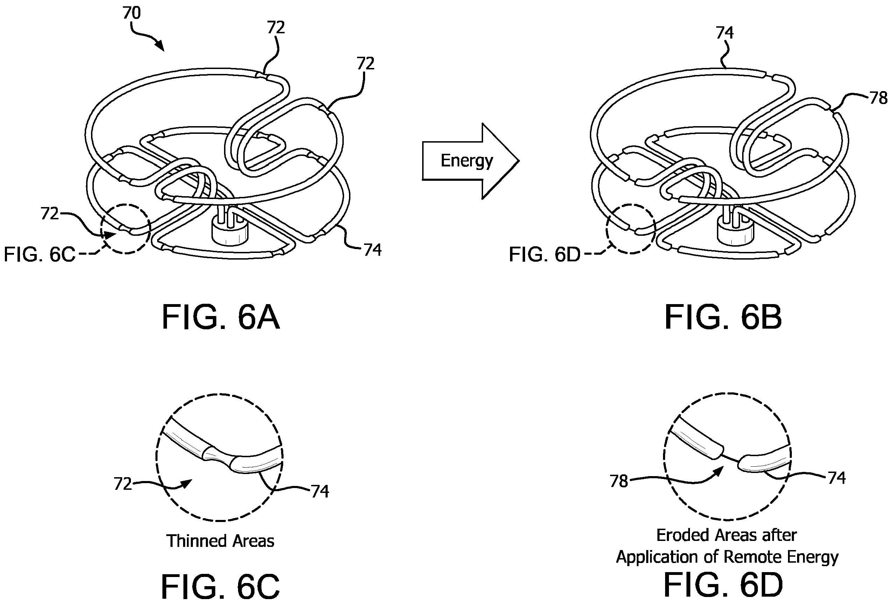

A second, non-limiting example of rendering a medical device conformable is depicted generally in FIG. 6A. FIG. 6A depicts an occluder 70 that may be formed of a drawn filled tube having an electrically conductive core and an outer surface as described above. One or more thinned areas 72 may be provided on the frame 74 of the occluder 70. An enlarged view of a thinned area 72 on the frame 74 is shown in FIG. 6C.

The occluder 70 may be covered in whole or in part with a biocompatible material, such as, for example, expanded polytetrafluoroethylene (ePTFE). The occluder 70 may be implanted in a body, and, after a period of time, the occluder 70 may become ingrown and/or covered with tissue. After a sufficient ingrowth of tissue, the frame 74 of the occluder 70 is no longer needed, as the ingrown tissue will hold the occluder 70 and covering material in place. At any time, particularly after tissue ingrowth into the occluder 70, energy may be transmitted to the occluder frame 74 from a remote energy source to erode the thinned areas 72 and compromise the occluder frame 74. Compromising the frame 74 reduces the chance of long-term abrasion during the cardiac cycle. As shown in FIG. 6B, a thinned area has been electrochemically degraded. An enlarged view of the degraded frame is shown in FIG. 6D. Reference numeral 78 indicates where the thinned region has been degraded but the corrosion-resistant core remains. Purposefully fracturing the frame (or removing portions of the frame's outer surface that contribute to its stiffness) permits the device 70 to be flexible and conformable.

In a further embodiment, a medical device may be formed of an electrochemically degrading material such that the entirety of the medical device is degraded or substantially degraded when an electrolytic cell is formed. For instance, the elements forming the medical device may be tapered and/or otherwise designed so that the degradation of the medical device proceeds in a predictable and orderly manner such that a complete dissolution of the medical device or a nearly complete dissolution of the medical device occurs. Any portion of the medical device that is not completely dissolved may be passed through the digestive tract. As described above, a corrosion-resistant component may be embedded or otherwise incorporated in the medical device to maintain electrical connection between disparate elements of the degrading structure until complete or sufficient dissolution of the target sections has been accomplished.

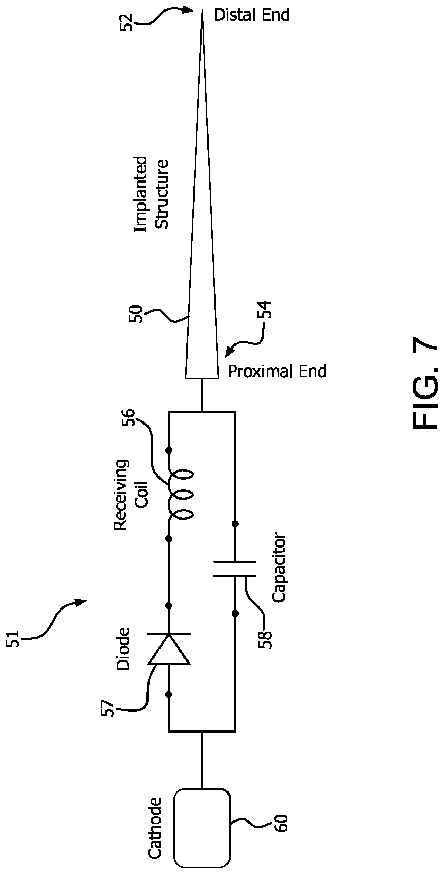

FIG. 7 depicts an implantable structure 50 formed of an electrochemically degrading material connected to an electrochemical cell 51 that includes a receiving coil 56, a diode 57, a capacitor 58, and a cathode 60. When energy is applied to the electrochemical cell 51, the implantable medical device 50 begins to degrade. Because the distal end 52 of the implantable device 50 contains the least amount of degradable material, it is the first region of the implantable device 50 to dissolve completely. More proximal regions are characterized by increasing areas or amounts of electrochemical degrading material, and as a result, complete dissolution of the implantable device 50 proceeds from the distal end 52 to the proximal end 54. It is to be noted that if a proximal region dissolves more quickly than a distal region, the electrical connection to the more distal regions would be lost and the electrolytic degradation of these distal regions would be incomplete. The result of such an incomplete degradation may be that the distal regions not completely degraded are too large to pass naturally through the digestive tract and surgery may be required to retrieve the non-degraded portion of the medical device. For at least this reason, it is desirable to design an implantable medical device so that the dissolution of the device may proceed in a predictable, controlled, and complete manner. As described above, a corrosion-resistant component may also facilitate this by maintaining electrical connection between disparate elements of the degrading structure until complete or sufficient degradation of the target sections has been accomplished.

In yet another embodiment, a voltage may be maintained on a medical device in order to prevent its degradation until a time when the medical device is no longer needed. Once the medical device has served its purpose, the voltage potential is removed and the medical device begins degrading. In at least one embodiment, the medical device dissolves completely.

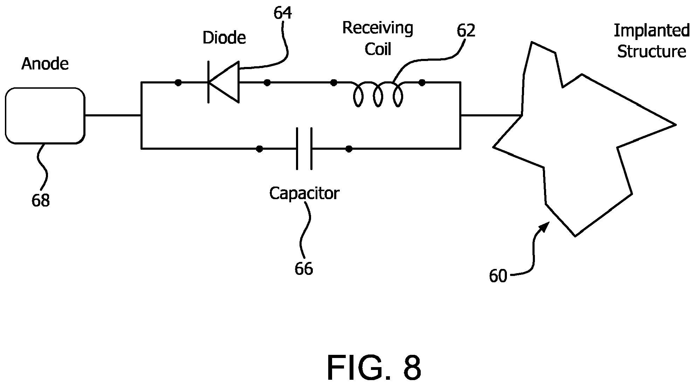

FIG. 8 schematically depicts such a medical device. The medical device 60 is connected to a receiving coil 62, a rectifier 64 (diode), a capacitor 66, and an anode electrode 68. The medical device 60 may be fabricated from a material that, when implanted, would naturally dissolve in the body over time. Non-limiting examples of such a material include iron and magnesium. The anode 68 may be fabricated from a conductive material, such as graphite, gold, iridium, or platinum, which provides an electrical connection to surrounding body tissue and fluids but which has a relatively high standard electrode potential, and is therefore relatively immune to corrosion. As long as energy is transmitted to the receiving coil 62, the voltage present maintains the anode 68 at a positive potential with respect to the medical device 60 and prevents corrosion of the medical device 60. When energy is no longer transmitted to the receiving coil 62, the potential difference is no longer present, and the medical device 60 begins to corrode. The receiving coil 62 and adjacent electrical connections may be fabricated from the same material as the medical device 60 so that they will also dissolve once the excitation energy has been removed.

It is to be noted that although the inventions described above are with reference to specific medical devices (e.g., stent devices, occluders, and embolic filters), it is to be appreciated that any medical device that contains a cathode, an anode region, is capable of receiving a wireless transmission from an external transmitter device, and contains at least one region that may be subjected to electrolytic degradation may be used and is considered to be within the purview of the invention. The devices described herein are exemplary in nature and are not meant to be limiting.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims and their equivalents.

Further examples and embodiments are provided in the following clauses:
1. An implantable device having a cathode region, a sacrificial anode region, and an antenna region, said implantable device comprising: a first configuration, and a second configuration wherein electrolytic degradation of said sacrificial anode region transforms said implantable device from said first configuration to said second configuration, and wherein said electrolytic degradation is initiated by the formation of an electrolytic cell, said electrolytic cell being formed when said antenna region remotely receives electrical energy from an external transmitter device.
2. The implantable device of clause 1, wherein said first configuration is an anchored configuration wherein said implantable device is anchored to a lumen wall, and wherein said second configuration is a non-anchored configuration in which said implantable device may be removed.
3. The implantable device of clause 1, wherein said first configuration is a first diameter of said implantable device, wherein said second configuration is a second diameter of said implantable device, and wherein said first diameter is less than said second diameter.
4. The implantable device of clause 3, wherein said second diameter anchors said implantable device in a lumen.
5. The implantable device of clause 1, further comprising at least one predetermined failure region susceptible to said electrolytic degradation.
6. The implantable device of clause 1, wherein said electrolytic degradation precipitates absorption of said implantable device.
7. An implantable device having a cathode region, a sacrificial anode region, and a piezoelectric receiver region, said implantable device comprising: a first configuration, and a second configuration wherein electrolytic degradation of said sacrificial anode region transforms said implantable device from said first configuration to said configuration, and wherein said electrolytic degradation is initiated by the formation of a electrolytic cell, said electrolytic cell being formed when said piezoelectric receiver region receives acoustic energy from an external transmitter device and converts the acoustic energy to electrical energy.
8. The implantable device of clause 7, wherein said first configuration is an anchored configuration wherein said implantable device is anchored to a lumen wall, and wherein said second configuration is a non-anchored configuration in which said implantable device may be removed.
9. The implantable device of clause 7, wherein said first configuration is a first diameter of said implantable device, and wherein said second configuration is a second diameter of said implantable device, wherein said first diameter is less than said second diameter.
10. The implantable device of clause 9, wherein said second diameter anchors said implantable device in a lumen.
11. The implantable device of clause 7, further comprising at least one predetermined failure region susceptible to said electrolytic degradation.
12. The implantable device of clause 7, wherein said electrolytic degradation precipitates absorption of said implantable device.
13. A method for remotely reconfiguring an implantable device comprising a sacrificial anode region, a cathode region, and an antenna region, said method comprising: receiving electrical energy from an external transmitter device to form a electrolytic cell and cause electrolytic degradation of said sacrificial anode region, wherein said electrolytic degradation transforms said implantable device from a first configuration to a second configuration.
14. The method of clause 13, wherein said first configuration is an anchored configuration wherein said implantable device is anchored to a lumen wall, and wherein said second configuration is a non-anchored configuration in which said implantable device may be removed.
15. The method of clause 13, wherein said implantable device is a stent comprising at least one region of variable diameter, and wherein said first configuration is a first diameter of said at least one region of variable diameter and said second configuration is a second diameter of said at least one region of variable diameter.
16. The method of clause 13, wherein said first configuration is a first diameter of said implantable device, and wherein said second configuration is a second diameter of said implantable device, and wherein said first diameter is less than said second diameter and said second diameter anchors said implantable device in a lumen.
17. The method of clause 13, wherein said first configuration is an anchored configuration wherein said implantable device is anchored to a lumen wall, and wherein said second configuration is a non-anchored configuration in which said implantable device may be removed.
18. The method of clause 13, further comprising at least one predetermined failure region susceptible to said electrolytic degradation.
19. An implantable device comprising: a cathode region and a sacrificial anode region between which an electrolytic cell is formed when an antenna region remotely receives energy from an external transmitter device, wherein electrolytic degradation of said sacrificial anode region occurs upon the formation of said electrolytic cell and transforms said implantable device from a first configuration to a second configuration.
20. The implantable device of clause 19, wherein said first configuration is an anchored configuration wherein said implantable device is anchored to a lumen wall, wherein said second configuration is a non-anchored configuration in which said implantable device may be removed.
21. The implantable device of clause 19, wherein said implantable device is a stent comprising at least one region of variable diameter, wherein said first configuration is a first diameter of said at least one region of variable diameter and said second configuration is a second diameter of said at least one region of variable diameter.
22. The implantable device of clause 19, wherein said implantable device has a first configuration as a vascular filter, wherein said first configuration of said implantable device positions emboli blocking elements in a blood stream and said second configuration removes said emboli blocking elements from the bloodstream.
23. The implantable device of clause 19, further comprising a rectification circuit adjacent to said antenna region to convert said electrical energy to direct current voltage.
24. The implantable device of clause 19, wherein said failure of said sacrificial anode region precipitates absorption of said implantable device.
25. The implantable device of clause 19, wherein said failure of said sacrificial anode region de-anchors said implantable device.
26. The implantable device of clause 19, wherein said sacrificial anode region comprises a narrowed region of a structural member of said device.
27. The implantable device of clause 19, wherein said sacrificial anode region comprises an electrically uninsulated subsection of a structural member of said device.
28. An implantable device comprising a cathode region, an anode region, and an antenna region, said implantable device comprising: a first configuration, and a second configuration wherein degradation of said cathode region transforms said implantable device from said first configuration to said second configuration, wherein said degradation of said cathode region is prohibited by the formation of an electrolytic cell causing active impressed current cathodic protection.
29. The implantable device of clause 28, wherein said first configuration is an anchored configuration wherein said implantable device is anchored to a lumen wall, and wherein said second configuration is a non-anchored configuration in which said implantable device may be removed.
30. The implantable device of clause 28, wherein said first configuration is a first diameter of said implantable device, and wherein said second configuration is a second diameter of said implantable device, wherein said first diameter is less than said second diameter.
31. The implantable device of clause 29, wherein said second diameter anchors said implantable device in a lumen.
32. A method of selectively degrading at least one predetermined portion of a conductive implantable device, said method comprising: remotely generating a voltage in said implantable device by coupling said implantable device to an electromagnetic field; converting said induced voltage to direct current voltage and forming an electrolytic cell; wherein structural failure of said implantable device at said at least one predetermined portion occurs upon the formation of the electrolytic cell, and wherein said structural failure transfigures said implantable device from a first configuration to a second configuration.
33. An implantable device comprising: a frame comprising an electrically conductive, corrosion resistant core and an electrochemically degradable outer surface, wherein energy transmitted to said frame degrades said outer surface and compromises the structural integrity of said frame.
34. The implantable device of clause 33, wherein said frame has at least one thinned region with a diameter smaller than a diameter of said frame.
35. An implantable device having a cathode region, a sacrificial anode region, and an antenna region, said implantable device comprising: a first configuration, and a second configuration wherein electrolytic degradation of said sacrificial anode region transforms said implantable device from said first configuration to said second configuration, and wherein electrical energy received by said antenna region from a remote transmitter is stored in said device, and wherein when said stored energy reaches a predetermined threshold, said stored energy is released to form an electrolytic cell which initiates said electrolytic degradation.

## Claims

1. An implantable device comprising:
a frame including an electrically conductive, corrosion resistant core and an electrochemically degradable outer surface,
wherein energy transmitted to the frame degrades the outer surface and compromises the structural integrity of the frame.

2. The implantable device of claim 1, wherein the frame has at least one thinned region with a diameter that is smaller than a diameter of the frame.

3. The implantable device of either claim 1 or claim 2, further comprising an antenna region operable to remotely receive the energy from an external transmitter.

4. The implantable device of any of the preceding claims, wherein the frame is configured such that energy transmitted to the frame degrades the outer surface of the frame by formation of an electrolytic cell.

5. The implantable device of claim 4, wherein the frame is configured such that formation of the electrolytic cell initiates degradation of the frame.

6. The implantable device of claim 5, wherein the degradation of the frame is configured to precipitate absorption of the implantable device when implanted in a body of a patient.

7. The implantable device of any of the preceding claims, wherein the frame includes an anchoring portion configured to anchor the implantable device in a bodily lumen.

8. The implantable device of claim 7, wherein the anchoring portion is configured to de-anchor from the bodily lumen then the structural integrity of the frame is compromised.

9. The implantable device of either claim 7 or claim 8, wherein the is insulated about the anchoring portion except at a sacrificial region.

10. The implantable device of any one of claims 7-9, wherein the anchoring portion includes spring force anchor wings.

11. The implantable device of claim 10, wherein the spring force anchor wings are operable to apply a spring force against tissue forming the bodily lumen.

12. The implantable device of claim 11, wherein the anchoring portion is configured to reduce the spring force when the sacrificial region fails.

13. The implantable device of any of the preceding claims, wherein the frame is operable to break when the structural integrity of the frame is compromised.

14. The implantable device of any of the preceding claims, wherein the frame is operable to break into at least two portions when the outer surface is degraded.
